# EUROPEAN PATENT SPECIFICATION

(11) **EP 4 551 123 B1**
(45) Date of publication and mention of the grant of the patent: **06.05.2026**
(21) Application number: 23753959.8
(22) Date of filing: 07.07.2023
(51) Int. Cl.: A61B 90/00, A61B 8/08, A61N 7/02, G06T 7/30, A61B 8/00, A61N 7/00

(54) **SYSTEM FOR REGISTERING IMAGES OBTAINED USING DIFFERENT IMAGING MODALITIES**
SYSTEM ZUR REGISTRIERUNG VON MITHILFE VERSCHIEDENER BILDGEBUNGSMODALITÄTEN ERHALTENEN BILDERN
SYSTÈME D'ENREGISTREMENT D'IMAGES OBTENUES À L'AIDE DE DIFFÉRENTES MODALITÉS D'IMAGERIE

(30) Priority: 07.07.2022 US 202263359076 P
(43) Date of publication of application: 14.05.2025
(73) Proprietor: Insightec Ltd., 3912001 Tirat-Carmel (IL)
(72) Inventor: BROKMAN, Omer, 3912001 Tirat Carmel (IL); LEVY, Yoav, 3912001 Tirat Carmel (IL); NAFTALIS, Netanel, 3912001 Tirat Carmel (IL)
(74) Representative: Dennemeyer & Associates S.A.
(86) International application number: PCT/IB2023/000391
(87) International publication number: WO 2024/009143

(56) References cited:
- EP-A1- 2 998 932
- WO-A1-2016/170427
- WO-A1-2018/026738
- WO-A2-2017/060865
- WO-A2-2021/123905

## Description

### TECHNICAL FIELD

The present disclosure relates to imaging, and, more specifically, to systems and methods for registering images and/or verifying image registrations obtained using various imaging modalities.

### BACKGROUND

Medical imaging of internal organs provides important anatomic and diagnostic information, which medical personnel can employ to make therapeutic decisions. Medical images can be acquired using various non-invasive imaging procedures, such as computed topography (CT), magnetic resonance imaging (MRI), or ultrasound imaging. A CT system transmits x-rays through an anatomic site of interest, and based on the attenuation coefficients of the x-rays, cross-sectional images ("slices") of the object can be reconstructed. As a result, CT scans are well suited to viewing details of bony structures, diagnosing diseases of the lung and chest, and detecting cancers. Advantages of CT imaging include, for example, a short scan time for a complete scan (typically less than five minutes), low cost (about half the price of an MRI apparatus), the ability to accurately outline bony tissue inside the body, fewer motion artifacts due to fast imaging speeds (each scan time is less than 30 seconds), etc. CT systems, however, irradiate the patient and pose consequent risk; for this reason, CT scans are not recommended for pregnant women or children.

Ultrasound imaging, which involves passing high-frequency acoustic waves through the body, is another widely used technique. Ultrasound waves penetrate well through soft tissues and, due to their short wavelengths, can be focused to spots with dimensions of a few millimeters. In a typical ultrasound examination, a transducer probe is placed directly on the skin or inside a body opening. A thin layer of gel may be applied to the skin to provide direct contact between the transducer probe and skin and thereby allow efficient transfer of ultrasound energy into the body. An ultrasound image of internal anatomic structures can be constructed from the waves reflected by those structures - in particular, for example, from the amplitudes and phases of the reflection signals and/or the time it takes for the ultrasound waves to travel through the body. Because ultrasound images are captured in real-time, they can also show movement of the body's internal organs as well as blood flowing through the blood vessels. In addition, ultrasound imaging offers high temporal resolution, high sensitivity to acoustic scatterers (such as calcifications and gas bubbles), excellent visualization, low cost, portability, and no ionizing radiation exposure, and is thus generally considered safe even for pregnant women and children. Acoustic imaging can also be effective for real-time mapping of cavitation events (which occur during ultrasound therapy) but cannot accurately identify soft tissue contours through the skull and other bony structure, which introduces shifts and inaccuracies.

MRI is still another imaging modality used to visualize internal tissue. MRI can be used in conjunction with therapeutic (as opposed to imaging) ultrasound to guide the ultrasound focus during therapy as further described below. In brief, MRI involves placing a patient into a homogeneous static magnetic field, thus aligning the spins of hydrogen nuclei in the tissue. Then, by applying a radiofrequency (RF) electromagnetic pulse of the right frequency (the "resonance frequency"), the spins may be flipped, temporarily destroying the alignment and inducing a response signal. Different tissues produce different response signals, resulting in a contrast among these tissues in MR images. Because the resonance frequency and the frequency of the response signal depend on the magnetic field strength, the origin and frequency of the response signal can be controlled by superposing magnetic gradient fields onto the homogeneous field to render the field strength dependent on position. By using time-varying gradient fields, MRI "scans" of the tissue can be obtained.

Many MRI protocols utilize time-dependent gradients in two or three mutually perpendicular directions. The relative strengths and timing of the gradient fields and RF pulses are specified in a pulse sequence. Time-dependent magnetic field gradients may be exploited, in combination with the tissue dependence of the MRI response signal, to visualize, for example, a brain tumor, and determine its location relative to the patient's skull. MRI has advantages including multi-planar imaging capability (without moving the patient), high signal-to-noise ratio, high sensitivity to subtle changes in soft tissue morphology and function, and no radiation exposure. But MRI suffers from its sensitivity to patient movement due to the long scan time (typically between 30 minutes to a few hours), lower-resolution images of bony structures, and interference from the operation of other RF devices. MRI is also less effective for real-time cavitation mapping.

Because each imaging technique has its own strengths and weaknesses and may provide different types of information, it may be advantageous in practice to combine different imaging techniques. For example, combining a CT scan with MRI can provide good details about bony structures as well as subtle differences between soft tissues. A combination of MRI and ultrasound imaging has been shown to provide additional diagnostic information for better diagnosis in intraoperative neurosurgical applications and breast biopsy guidance. Further, because ultrasound energy can be employed therapeutically - e.g., to heat and ablate diseased (e.g., cancerous) tissue without causing significant damage to surrounding healthy tissue - the combination of MRI and ultrasound provides imaging capability during therapeutic medical procedures.

An ultrasound focusing system generally includes an acoustic transducer surface, or an array of transducer surfaces, to generate one or more ultrasound beams. In transducer arrays, the individual surfaces, or "elements," are typically individually controllable - i.e., their vibration phases and/or amplitudes can be set independently of one another - allowing the beam to be steered in a desired direction and focused at a desired distance. The ultrasound system often also includes receiving elements, integrated into the transducer array or provided in form of a separate detector, that help monitor ultrasound-based treatment. For example, the receiving elements may detect ultrasound reflected by interfaces between the transducer and the target tissue, which may result from air bubbles on the skin that need to be removed to avoid skin burns. The receiving elements may also be used to detect cavitation in overheated tissues (i.e., the formation of cavities due to the collapse of bubbles formed in the liquid of the tissue).

A focused ultrasound transducer system may include MR tracking coils or other markers for determining the transducer position and orientation relative to the target tissue (such as a tumor) in the MR image. Based on computations of the required transducer element phases and amplitudes, the transducer array is then driven so as to focus ultrasound at the target. The ultrasound focus itself may be visualized using MRI or acoustic resonance force imaging (ARFI), and such visualization may be used to adjust the focus position. These methods are generally referred to as magnetic-resonance-guided focusing of ultrasound (MRgFUS).

To successfully integrate two or more imaging systems and/or combine information provided by distinct systems, it is necessary to register image data that may be obtained in different imaging coordinate systems. Conventional approaches to registration typically involve complex computational procedures and may not provide sufficient accuracy for therapeutic purposes. For example, when using acoustic imaging for real-time cavitation mapping in conjunction with MRI to image soft tissues, there may be no useful features that both modalities can accurately identify in order to facilitate registration.
WO 2018/026738 A1 relates to ultrasound systems and methods that apply ultrasound to facilitate delivery of drugs to the brain.
WO 2021/123905 A2 relates to generating microbubbles in an anatomic target region with an ultrasound transducer.

### SUMMARY

At least one of these objects is solved by the features of the independent claim 1.

Embodiments of the present disclosure provide systems and methods for registering different imaging modalities to a common coordinate reference frame or to each other. Transient or clinically harmless tissue disruptions are created at a known anatomic location and are visualized by both imaging systems, enabling their registration. In various embodiments, one of the imaging systems is an ultrasound system capable of delivering both imaging and therapeutic acoustic energy. In its imaging mode, the ultrasound system senses reflections of acoustic imaging pulses directed at a target location. Operating the ultrasound system in therapy mode enables targeted delivery of high-amplitude, highly focused ultrasound bursts that create a tissue effect observable by the second imaging system, e.g., an MR system. The tissue effect may be transient, e.g., minor and reversible tissue damage, or may involve longer-lasting but minuscule tissue damage. In either case, the resulting micro-bleeding may be detected by MRI. The tissue effect may be produced by local cavitation events in the target tissue caused by acoustic energy deposition.

In other embodiments, the tissue effect is a detectable change in tissue permeability, such as opening of the blood-brain barrier (BBB) to permit traversal of molecules that would otherwise be unable to pass through. Such molecules may be detectable by the second imaging system, e.g., they may accumulate detectably across the BBB where it has been sufficiently disrupted. For example, MR contrast agents may include gadolinium ion, which is readily detectable in MR images. Ultrasound contrast agents can include micron-scale bubbles (microbubbles), nanometer-scale bubbles (nanobubbles), phase-shift nanodroplets, gas vesicles, echogenic liposomes and/or polymeric nanoparticles; *see, e.g.,* Helfield et al., "Acoustically-Stimulated Nanobubbles: Opportunities in Medical Ultrasound Imaging and Therapy," Front. Phys., 07 May 2021. A contrast agent may additionally or alternatively be a liquid droplet/phase-change contrast agent or modified liposomes or other lipid vesicles conjugated with conventional contrast agents. Liposomes and other vesicles can be filled with or attached to drugs, so it is possible not only to image in two modalities but to also treat at the same time.

The precise location of the tissue effect may be established by the ultrasound system, which detects - using dedicated receiving transducer elements or the same elements that transmitted the acoustic dose but operated in a receive mode - the acoustic reflection from collapsing microbubbles due to the cavitation event. The ultrasound system may produce an image of the target region or, more typically, may simply determine the location of the cavitation event based on time-of-flight analysis of the received reflection signal. Acoustic reflections from cavitation events have characteristic signal features that enable their isolation from other reflection signals.

Once the location of the cavitation event has been established using the ultrasound system and the resulting and co-located tissue disruption identified by the other imaging system, the two systems may be registered in a common coordinate reference frame. A coordinate transformation may be established to relate the spatial reference frame of each imaging system to the other.

Although the ensuing discussion focuses primarily on ultrasound and MRI systems, it should be understood that the present disclosure is useful with any imaging systems that can detect co-located internal markers. Further, it is not necessary that one of the imaging systems actually create the markers, though this may simplify their localization. For example, a third system may induce tissue disruption at a known internal location. When the tissue disruption (or a consequence thereof, such as micro-bleeding or detectable permeability) is detected and spatially localized by both systems, they may be registered based on the known internal location.

As used herein, the terms "approximately," "roughly," and "substantially" mean ±10%, and in some embodiments, ±5%. Reference throughout this specification to "one example," "an example," "one embodiment," or "an embodiment" means that a particular feature, structure, or characteristic described in connection with the example is included in at least one example of the present technology. Thus, the occurrences of the phrases "in one example," "in an example," "one embodiment," or "an embodiment" in various places throughout this specification are not necessarily all referring to the same example. Furthermore, the particular features, structures, routines, steps, or characteristics may be combined in any suitable manner in one or more examples of the technology. The headings provided herein are for convenience only and are not intended to limit or interpret the scope or meaning of the claimed technology.

### BRIEF DESCRIPTION OF THE DRAWINGS

In the drawings, like reference characters generally refer to the same parts throughout the different views. Also, the drawings are not necessarily to scale, with an emphasis instead generally being placed upon illustrating the principles of the disclosure. In the following description, various embodiments of the present disclosure are described with reference to the following drawings, in which:
FIG. 1A schematically depicts an exemplary MRI system in accordance with various embodiments of the current disclosure;
FIG. 1B schematically depicts an exemplary CT system in accordance with various embodiments of the current disclosure;
FIG. 1C schematically depicts an exemplary ultrasound system in accordance with various embodiments of the current disclosure;
FIG. 2A schematically illustrates two imaging systems operating in registration.
FIG. 2B schematically illustrates two imaging systems operating out of registration.
FIGS. 2C and 2D schematically illustrate the effect of aberration in deflecting the ultrasound focus away from an intended target location.
FIG. 3 shows a flowchart of an example method for registering imaging systems while providing focused ultrasound to a target region, according to some embodiments.

### DETAILED DESCRIPTION

### Representative Imaging Environments

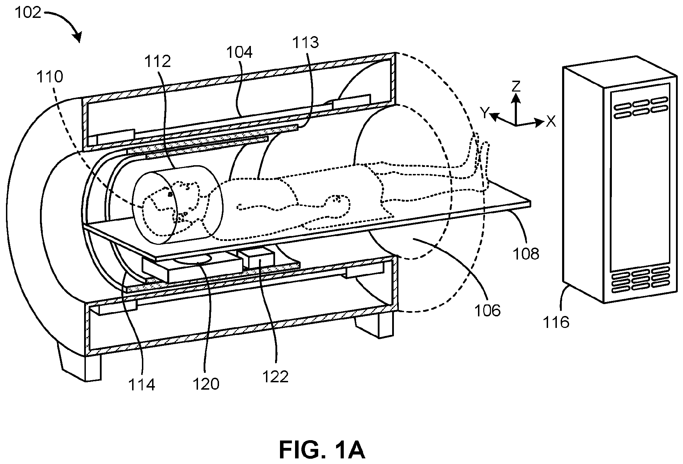

FIG. 1A illustrates an exemplary MRI apparatus 102. The apparatus 102 may include a cylindrical electromagnet 104, which generates the requisite static magnetic field within a bore 106 of the electromagnet 104. During medical procedures, a patient is placed inside the bore 106 on a movable support table 108. A region of interest 110 within the patient (e.g., the patient's head) may be positioned within an imaging region 112 wherein the electromagnet 104 generates a substantially homogeneous field. A set of cylindrical magnetic field gradient coils 113 may also be provided within the bore 106 and surrounding the patient. The gradient coils 113 generate magnetic field gradients of predetermined magnitudes, at predetermined times, and in three mutually orthogonal directions. With the field gradients, different spatial locations can be associated with different precession frequencies, thereby giving an MR image its spatial resolution. An RF transmitter coil 114 surrounding the imaging region 112 emits RF pulses into the imaging region 112 to cause the patient's tissues to emit magnetic-resonance (MR) response signals. Raw MR response signals are sensed by the RF coil 114 and passed to an MR controller 116 that then computes an MR image, which may be displayed to the user. Alternatively, separate MR transmitter and receiver coils may be used. Images acquired using the MRI apparatus 102 may provide radiologists and physicians with a visual contrast between different tissues and detailed internal views of a patient's anatomy that cannot be visualized with conventional x-ray technology.

The MRI controller 116 may control the pulse sequence, i.e., the relative timing and strengths of the magnetic field gradients and the RF excitation pulses and response detection periods. The MR response signals are amplified, conditioned, and digitized into raw data using a conventional image-processing system, and further transformed into arrays of image data by methods known to those of ordinary skill in the art. Based on the image data, a treatment region (e.g., a tumor) is identified. The image-processing system may be part of the MRI controller 116, or may be a separate device (e.g., a general-purpose computer containing image-processing software) in communication with the MRI controller 116. In some embodiments, one or more ultrasound systems 120 or one or more sensors 122 are displaced within the bore 106 of the MRI apparatus 102 as further described below.

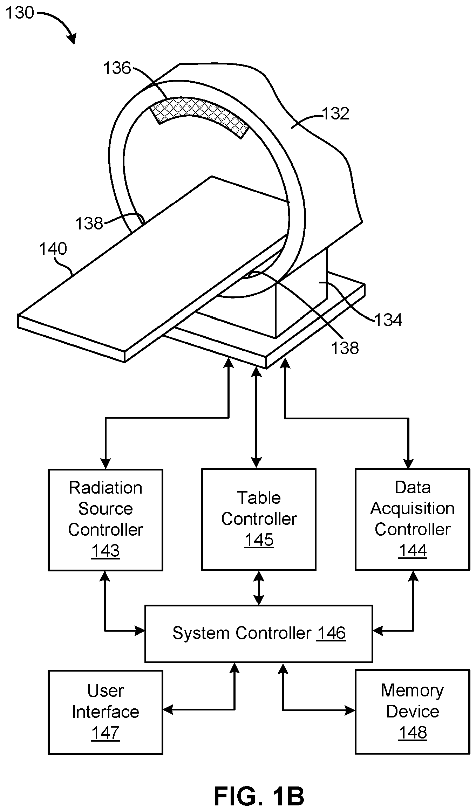

FIG. 1B illustrates an exemplary CT system 130 in accordance with embodiments of the present disclosure. The CT system 130 includes a scanner 132 having a support structure 134, an x-ray source 136, and an x-ray detector 138 on the same or opposite side of the source 136. The scanner 132 is configured to receive a table 140 for a patient to be scanned. The table 140 can be moved through an aperture in the scanner 132 to appropriately position the patient in an imaging volume or plane scanned during imaging sequences. The CT system 130 further includes a radiation source controller 143, a data acquisition controller 144, and a table controller 145. The radiation source controller 143 regulates timing for discharges of x-ray radiation from the x-ray source 136 toward the patient. The data acquisition controller 144 communicates with the x-ray detector 138 to measure the x-ray intensity data transmitted through or reflected from the patient. The acquired intensity data is processed by the data acquisition controller 144 or a system controller 146 for CT image reconstruction. The table controller 145 serves to adjust the table position during or between imaging sequences, depending upon the employed imaging protocol. The controllers 143, 144, 145, 146 may be separate units or may be integrated as a single unit.

In addition, the system controller 146 may be coupled to a user interface 147 and a memory device 148. The user interface 147 may be integrated with the system controller 146, and may generally include a user workstation for initiating imaging sequences, controlling such sequences, and manipulating data acquired during imaging sequences. The memory devices 148 may be local to the imaging system, or partially or completely remote from the system, and may be configured to receive raw, partially processed or fully processed data for CT image reconstruction.

During a typical CT scan, the x-ray scanner 132 rotates around the patient with a predetermined speed, and the x-ray source 136 emits narrow beams of x-rays through the body. The x-ray intensities transmitted through or reflected from the patient at various angles with respect to the patient's body are measured by the detector 138. A two-dimensional tomographic image (i.e., a "slice") of the patient can be created based on the detected beam angles and intensities of the x-rays. Multiple slices obtained at different angles can then be processed to reconstruct a CT image. In some embodiments, the CT image of the patient is acquired at a planning stage, i.e., prior to a thermal treatment, to allow time for the treatment plan to be prepared.

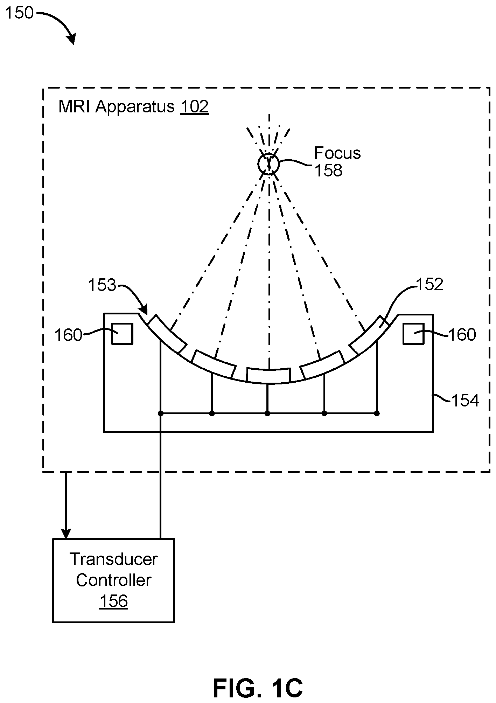

FIG. 1C illustrates an exemplary ultrasound system 150 (e.g., corresponding to 120, FIG. 1A) in accordance with some embodiments of the present disclosure. As shown, the ultrasound system includes a plurality of ultrasound transducer elements 152, which are arranged in an array 153 at the surface of a housing 154. The array may comprise a single row or a matrix of transducer elements 152. In alternative embodiments, the transducer elements 152 may be arranged without coordination, i.e., they need not be spaced regularly or arranged in a regular pattern. The array may have a curved (e.g., spherical or parabolic) shape, as illustrated, or may include one or more planar or otherwise shaped sections. Its dimensions may vary, depending on the application, between millimeters and tens of centimeters. The transducer elements 152 may be piezoelectric ceramic elements. Piezo-composite materials, or generally any materials capable of converting electrical energy to acoustic energy, may also be used. To damp the mechanical coupling between the elements 152, they may be mounted on the housing 154 using silicone rubber or any other suitable damping material.

The transducer elements 152 are separately controllable, i.e., they are each capable of emitting ultrasound waves at amplitudes and/or phases that are independent of the amplitudes and/or phases of the other transducers. A transducer controller 156 serves to drive the transducer elements 152. For n transducer elements, the controller 156 may contain n control circuits each comprising an amplifier and a phase delay circuit, each control circuit driving one of the transducer elements. The controller 156 may split an RF input signal, typically in the range from 0.1 MHz to 4 MHz, into n channels for the n control circuits. It may be configured to drive the individual transducer elements 152 of the array at the same frequency, but at different phases and different amplitudes so that they collectively produce a focused ultrasound beam. The transducer controller 156 desirably provides computational functionality, which may be implemented in software, hardware, firmware, hardwiring, or any combination thereof, to compute the required phases and amplitudes for a desired focus location 158 (e.g., corresponding to 110, FIG. 1A). In general, the controller 156 may include several separable apparatus, such as a frequency generator, a beamformer containing the amplifier and phase delay circuitry, and a computer (e.g., a general-purpose computer) performing the computations and communicating the phases and amplitudes for the individual transducer elements 152 to the beamformer. Such systems are readily available or can be implemented without undue experimentation.

To perform ultrasound imaging, the controller 156 drives the transducer elements 152 to transmit acoustic signals into a region being imaged and to receive reflected signals from various structures and organs within the patient's body. By appropriately delaying the pulses applied to each transducer element 152, a focused ultrasound beam can be transmitted along a desired scan line. Acoustic signals reflected from a given point within the patient's body are received by the transducer elements 152 at different times. The transducer elements can then convert the received acoustic signals to electrical signals which are supplied to the beamformer. The delayed signals from each transducer element 152 are summed by the beamformer to provide a scanner signal that is a representation of the reflected energy level along a given scan line. This process is repeated for multiple scan lines to provide signals for generating an image of the prescribed region of the patient's body. Typically, the scan pattern is a sector scan, wherein the scan lines originate at the center of the ultrasound transducer and are directed at different angles. A linear, curvilinear or any other scan pattern can also be utilized.

Focused ultrasound can also be used therapeutically to treat internal body tissues within a patient, and as described below, therapeutic applications can complement imaging by creating markers visible to the imaging system. In particular, ultrasound waves may be used in applications involving ablation of tumors, thereby eliminating the need for invasive surgery, targeted drug delivery, control of the blood-brain barrier, lysing of clots, and other surgical procedures. During tumor ablation, focused ultrasound energy is deposited at sufficient levels to ablate tissue. This procedure is often called high-intensity focused ultrasound (HIFU) therapy and is described in detail in, for example, PCT Appl. No. WO 2021/123906, filed on December 18, 2020.

Alternatively, the transducer array 153 may be operated at high enough power to create cavitation at a target location. One such procedure is histotripsy, which involves the delivery of acoustic energy in the form of short (generally less than 50 µsec), high-amplitude pulses that deposit relatively low total energy at high peak pressure. This induces brief cavitation to mechanically rupture targeted tissue. Cavitation occurs when a sufficiently negative pressure is applied to tissue to cause microbubble formation from fluid vaporization and release of dissolved gas. Once formed, the microbubbles exhibit highly dynamic patterns of oscillation and inertial collapse that produce cellular and tissue disruption. Whereas lesioning applications such as HIFU are sensitive to the amount of energy reaching the target, histotripsy is more sensitive to the negative peak pressure.

Another therapeutic application of focused ultrasound involves tissue permeability enhancements in a target region. For example, the target tissue may normally be permeable to molecules having a size less than 400 Daltons, but the therapeutic agent may have a size of 1,000 Daltons or more and therefore is blocked from entering the target region. Upon application of ultrasound, tissue in or regulating access to the target region (e.g., the BBB) may be disrupted, and consequently, the permeability thereof may be increased. The permeability level and/or the size of the tissue region in which the permeability has been increased may depend on the intensity and/or duration of the ultrasound application. Accordingly, by adjusting ultrasound parameters, the tissue permeability of the target region can be increased to a desired degree to allow the therapeutic agent to penetrate and/or diffuse therein. The degree of tissue permeability may be monitored using a molecule or molecular complex having a known size (which is too large to penetrate the intact BBB) coupled to a visualization agent. The visualization agent may be a contrast agent, e.g., a fluorophore- or Gd-labeled liposome; see, e.g., U.S. Serial No. 63/322,617, filed on March 22, 2022 and entitled "Monitoring Tissue Permeability During Ultrasound Procedures". The molecule or molecular complex may be a liposome, a quantum dot, or a dextran. If the size of the molecule or molecular complex is similar to that of a therapeutic agent (e.g., an antibody or other biologic), detecting passage of the visualization agent indicates sufficient BBB opening to admit the therapeutic agent.

The ultrasound system 150 may be disposed within the bore 106 of the MRI apparatus 102 or placed in the vicinity of the MRI apparatus 102. The combined MRI-ultrasound system is capable of monitoring the application of ultrasound for treatment and/or safety purposes. To determine the relative positions of the ultrasound system 150 and MRI apparatus 102, the ultrasound system 150 may further include MR trackers 160 associated therewith, each arranged at a fixed position and orientation relative to the ultrasound system 150. The trackers 160 may, for example, be incorporated into or attached to the ultrasound system housing 154. If the relative positions and orientations of the MR trackers 160 and ultrasound system 150 are known, MR scans that include, in the resulting images, the MR trackers 160 implicitly reveal the location of the ultrasound system 150 in MRI coordinates (in the coordinate system of the MRI apparatus 102). To aid in relating the ultrasound coordinate system to the MRI coordinate system, in some embodiments, an MR image including at least a portion (e.g., some transducer elements) of the ultrasound system 150 is acquired. This MR image may be used in combination with the known spatial arrangement of the ultrasound transducer elements to determine the locations of the transducer elements (not all of which need be included in the acquired MR image) in the MRI coordinate system as further described below.

While penetrating the patient, ultrasound waves typically encounter multiple layers of tissues, e.g., bone, muscle, or fat, whose density and structure, and, consequently, ultrasound propagation properties differ. Due to inhomogeneities and anisotropies in the tissues, the ultrasound wave fronts are often distorted. Moreover, signals from different transducer elements may encounter different thicknesses and contours of materials, and possibly air-filled or liquid-filled pockets between transducer elements and the region to be imaged or treated, resulting in different phase shifts and attenuations. Knowledge of the structure, density, and/or thickness of the multi-layer tissue structures is thus important for compensating for these effects by appropriate phase shifts and amplification factors imposed on the transducer elements, and for avoiding deterioration of focusing properties. While MRI generally provides high-sensitivity images of soft tissue (e.g., the brain), the CT scan creates images with more details about bony structures (e.g., the skull). Accordingly, it may be beneficial to combine image information obtained from the MRI apparatus 102 and CT system 130. To do so, of course, requires registration of the images and, therefore, registration of the MRI and CT coordinate systems.

### Device Registration

The following disclosure describes registration of two or more imaging devices. It should be noted at the outset that an "imaging device" may be capable of applications other than imaging, e.g., an ultrasound system may be operated to perform various therapeutic applications as described above. References herein to imaging systems broadly connote such systems whether or not they are actually operated to create images. Furthermore, although the present discussion focuses on ultrasound and MR imaging, this is for illustration only. The disclosure is applicable to other imaging modalities such as CT, optical, and positron-emission tomography (PET), for example.

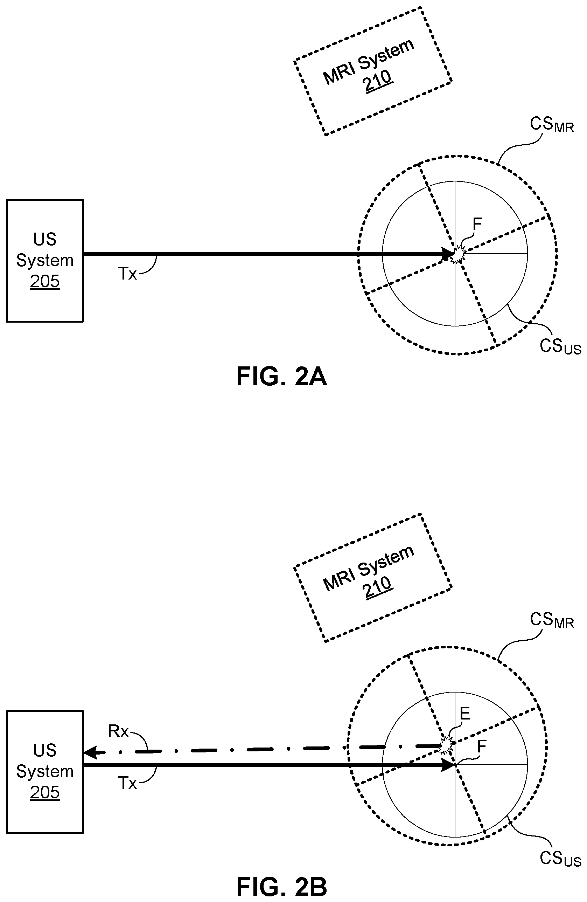

With reference to FIG. 2A, an ultrasound transducer system 205 (e.g., corresponding to system 150, FIG. 1C) has an associated coordinate system CS_{US} such that events that it records are located with respect to this coordinate system. An MRI system 210 (e.g., corresponding to system 102, FIG. 1A) has an associated coordinate system CS_{MR} such that events that it records are located with respect to this coordinate system. In operation, the ultrasound transducer system 205 is operated by a controller (e.g., controller 156, FIG. 1C) to produce, for example, a cavitation event by emitting ultrasound along the vector arrow Tx and focused at a target F (also referred to as a focus F). In practice, this would involve emissions from various spatially distributed transducer elements (e.g., elements 152, FIG. 1C) whose outputs converge at the target F, but which for illustration are shown as if emanating along a single path Tx. When the systems are in registration, as shown in FIG. 2A, the target F appears at the same point (e.g., the origin as illustrated) in each coordinate system CS_{US} and CS_{MR}.

Detectable events, such as cavitation, may be both induced and monitored by the ultrasound system 205 as described, for example, in U.S. Patent Publ. No. 2022/0043143. As described therein, one or more transient acoustic reflectors may be generated at or near the focus F by operating the ultrasound system 205 to emit high-power pulses that create cavitation events and produce microbubbles, which serve as transient acoustic reflectors. Subsequently, the ultrasound transducer system 205 is operated to generate a focus at the same location and to receive the signals reflected from the transient reflectors, either by the transducer elements themselves switched to operate in a receiving mode, by other transducer elements that did not emit the high-power pulses, or by one or more acoustic-signal detectors. As used herein, the term "transient reflector" refers to an acoustic reflector that dissipates or evolves with time during sonications. The term "imaging" includes this and similar localizing operations, and the localized event is an "image" for purposes hereof.

The location of the transient reflectors at the target F may be determined using a time-of-flight technique. Because the relative spatial locations of all ultrasound transducer elements are known in advance, the time of flight of the signal - i.e., the interval between ultrasound emission and detection of the reflection at each monitoring (receiving) transducer - may be used to locate the detected event in the coordinate system CS_{US}.

If the detectable event E at the target F produces not only transient reflectors but a tissue disruption at the same location and detectable by the MRI system 210, the coordinate systems CS_{US} and CS_{MR} may be registered by assigning the same location in each coordinate system to the target F (to the detectable event E). This procedure depends on accurate placement of the detectable event by the ultrasound system 205. If, as shown in FIG. 2B, the event E occurs at a location displaced from the target F (i.e., from where the ultrasound emissions are actually focused or thought to be focused), registration will be compromised; for this reason, it is important to ensure consistency between emission and reflection locations using a calibration procedure as described, for example, in the'3143 application.

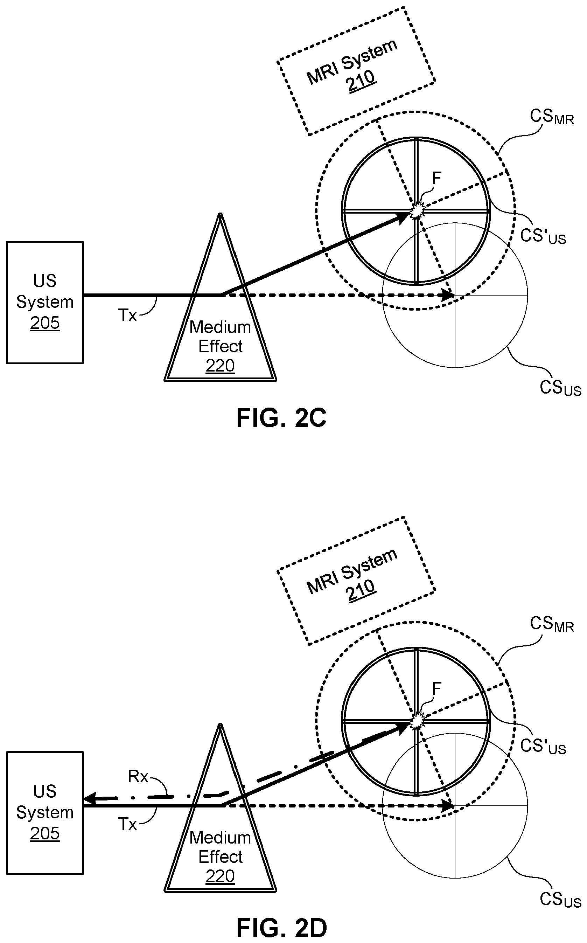

As is well known, tissue inhomogeneity may cause refraction of acoustic energy at the boundaries of tissue regions having different speeds of sound. Refraction may decrease constructive interference, and hence, the intensity of the acoustic energy at the focal zone, particularly when the acoustic energy passes through bone. Thus, inhomogeneous tissue structures may generate beam aberrations and refractions, which may distort or shift the focus. This is illustrated as a "medium effect" 220 in FIGS. 2C and 2D in the sense that tissue is the medium through which measured ultrasound signals pass. Because tissue homogeneities identically affect the transmitted ultrasound waves (Tx) and reflected ultrasound waves (Rx), as shown in FIG. 2D, even a perfectly calibrated ultrasound system 205 will not account for aberration-induced focus shifts. In effect, the ultrasound controller 156 does not "know" what effect the medium will have on the focus location.

Various techniques to correct aberration-induced distortion or shift have been developed; see, e.g., U.S. Patent Nos. 8,088,067 and 11,291,430 and U.S. Patent Publ. No. 2020/0085409. These techniques are important in treatment but not necessarily to registration of the ultrasound and MRI coordinate systems, so long as images generated by the MRI system are not subject to the same aberrations. Typically they will not be, since variations in tissue density affect neither the nuclear magnetic resonance phenomenon nor its electromagnetic detection. Registration between the true MRI coordinates CS_{MR} and the shifted ultrasound coordinates CS'_{US} is adequate in practice if the difference between the true and apparent ultrasound coordinates is small or if, as is ordinarily the case, the treatment procedure includes correction for aberration. In the latter case, so long as the correction is made for treatment, it is not usually necessary to apply this correction to registration as well, since the shifted coordinate system CS'_{US} is the one in which the treatment is done.

As explained above, the co-located tissue effects achieve visibility in both the ultrasound imaging system 205 and the MR imaging system 210. In one embodiment, the ultrasound transducer array is operated at high enough power to create cavitation at the focus F. One such procedure is histotripsy, in which acoustic energy is delivered in short (generally less than 50 µsec), high-amplitude pulses induce brief cavitation to mechanically rupture targeted tissue. Cavitation occurs when a sufficiently negative pressure is applied to tissue to cause microbubble formation from fluid vaporization and release of dissolved gas. See, e.g., Xu et al., "Histotripsy: the first noninvasive, non-ionizing, non-thermal ablation technique based on ultrasound," Int. J. Hyperthermia, 2021, 38(1): 561-575; Roberts, "Development and Translation of Histotripsy: Current Status and Future Directions," Curr. Opin. Urol., Jan. 2014, 24(1): 104-110. Once formed, the microbubbles exhibit highly dynamic patterns of oscillation and inertial collapse that produce cellular and tissue disruption, leading to small (e.g., 2 mm or less) bleed regions that heal quickly but can be detected in an MR image. See, e.g., Mitchell et al., "Detection of subarachnoid hemorrhage with magnetic resonance imaging," J. Neurol. Neurosurg. Psychiatry, 2001, 70:205-211. The gradient echo T2* sequence is known to exhibit good sensitivity to internal hemorrhage. Id. Other sequences, such as T2 and fluid attenuation inversion recovery (FLAIR), may also or instead be used. More generally, MRI images are complex and features therein may be detected in various ways - for example, an MRI image may be a map of the magnitudes of the MR response signals or a map of the signal phases; a feature may be detectable based on the magnitudes of pixels associated with the feature, the phases of pixels associated with the feature or phase differences with respect to a baseline image, or a combination of the foregoing.

Use of an ultrasound contrast agent (typically a dispersion of gas microbubbles) can reduce the required energy intensity and pressure needed for inducing cavitation. This, in turn, reduces the risk of excessive tissue damage. For example, in the presence of a contrast agent, the required peak focal pressure for histotripsy may be reduced from approximately 20 MP to 1 MP. U.S. Serial No. 16/378,945 describes administration systems for contrast agents including tailoring microbubble characteristics to particular applications. Similar systems may be used to administer MRI contrast agents. Alternatively, contrast agents may be administered parenterally by syringe or IV device.

In another embodiment, the MRI-detectable tissue disruption is permeability enhancement. The use of HIFU to enhance permeability across tissue barriers (e.g., the BBB) is well established; see, e.g., Burgess et al., "Drug delivery across the blood-brain barrier using focused ultrasound," Expert Opin. Drug Deliv., 2014, 11(5): 711-721. Permeability enhancements can be detected using an MRI contrast agent, such as nanoparticles including gadolinium (see Conti et al., "Empirical and Theoretical Characterization of the Diffusion Process of Different Gadolinium-Based Nanoparticles within the Brain Tissue after Ultrasound-Induced Permeabilization of the Blood-Brain Barrier," Contrast Media and Mol. Im., 2019, Article ID 6341545), fluorophores, or combinations thereof, often encapsulated in a liposome (see, e.g., Aryal et al., "MRI Monitoring and Quantification of Ultrasound-Mediated Delivery of Liposomes Dually Labeled with Gadolinium and Fluorophore through the Blood-Brain Barrier," Ultrasound Med. Biol., 2019, 45(7): 1733-1742).

MRI has been used, for example, to detect the presence of contrast agents within the brain extracellular space after ultrasound-induced BBB permeabilization. The MRI-detectable accumulation of contrast agent at the site of the ultrasound focus may be used in accordance herewith to register the ultrasound and MRI coordinate reference frames. More generally, the MRI-detectable molecule or molecular complex may be a liposome, a quantum dot, or a dextran. This approach avoids tissue damage, since the disruption is fully reversible without healing. Moreover, the MRI contrast agent may contain microbubbles or other species that reduce the threshold ultrasound intensity required to induce cavitation and consequent enhancement of tissue permeability as well as detectable reflection of the ultrasound signals. That is, a single contrast agent may provide both an acoustic and an MRI response. Alternatively, MRI and ultrasound contrast agents can be combined or otherwise administered together near the region of interest (or may be formulated to concentrate at the region of interest) in order to provide both ultrasound and MR visibility. The contrast agent may be administered (and/or the imaging system controlled) so that the contrast agent is present at the target region images are recorded.

Once the location of the target F has been established for both the ultrasound and MR imaging systems in their respective coordinate reference frames, the device controllers 146, 156 may communicate to share this information, and either of the device controllers 146, 156 may establish the coordinate transformation to relate the spatial reference frame of each imaging system to the other (or to another reference coordinate system) and communicate the parameters to the other device controller. This coordinate transformation may be, for example, in the form of a matrix or a system of linear equations. Images generated by both imaging systems may then be registered and combined into a single image. Similarly, features detected using the MRI system 210 but not resolvable by the ultrasound system 205 in its imaging mode may be used to guide therapeutic use of the ultrasound system 205.

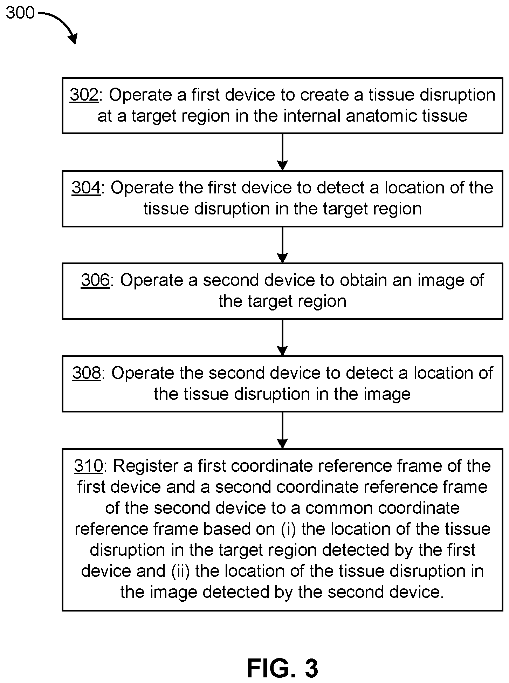

FIG. 3 shows a flowchart of an example method for registering imaging systems while providing focused ultrasound to a target region, according to some embodiments. The process may be governed by instructions that are stored in a computer memory or non-transitory computer readable storage medium. The instructions may be included in one or more programs stored in the non-transitory computer readable storage medium. When executed by one or more processors (e.g., 116; 146; 156; and/or a processor controlling 116, 146, and/or 156), the instructions cause the system to perform the process. The non-transitory computer readable storage medium may include one or more solid state storage devices (e.g., Flash memory), magnetic or optical disk storage devices, or other non-volatile memory devices. The instructions may include source code, assembly language code, object code, or any other instruction format that can be interpreted by one or more processors.

In operation 302, one or more processors (e.g., 116; 146; 156; and/or a processor controlling 116, 146, and/or 156) operates a first device (e.g., ultrasound system 150) to create a tissue disruption at a target region in internal anatomic tissue (e.g., 110).

In operation 304, the one or more processors operate the first device (e.g., transducer elements 152 configured to receive signals) to detect a location of the tissue disruption in the target region.

In operation 306, the one or more processors operate a second device (e.g., MRI apparatus 102) to obtain an image of the target region (e.g., 110).

In operation 308, the one or more processors operate the second device to detect a location of the tissue disruption in the image.

In operation 308, the one or more processors register a first coordinate reference frame of the first device and a second coordinate reference frame of the second device to a common coordinate reference frame based on (i) the location of the tissue disruption in the target region detected by the first device and (ii) the location of the tissue disruption in the image detected by the second device.

In some implementations, the one or more processors are configured to register the first and second coordinate reference frames to the common coordinate reference frame by assigning the same location (e.g., F in Figs. 2A-2D) in each of the first and second coordinate reference frames to the tissue disruption in the target region.

In some implementations, the one or more processors are configured to register the first and second coordinate reference frames to the common coordinate reference frame by establishing a coordinate transformation to relate one of the first and second coordinate reference frames to the other of the first and second coordinate reference frames. In some implementations, the one or more processors are configured to operate one of the first and second devices to communicate the coordinate transformation to the other of the first and second devices.

In some implementations, the tissue disruption is visualizable by MRI and/or computationally detectable in a T2 MR image or in a T2* MR image. In some implementations, the tissue disruption is a transient disruption.

In some implementations, the one or more processors are configured to operate the ultrasound transducer system to induce acoustic cavitation at the target region, and the tissue disruption is mechanically ruptured tissue caused by the acoustic cavitation.

In some implementations, the one or more processors are configured to (i) operate a source of ultrasound contrast agent including a suspension of microbubbles and (ii) cause the ultrasound contrast agent to be administered such that the ultrasound contrast agent reaches the target region before the first device is operated to create the tissue disruption at the target region.

In some implementations, the one or more processors are configured to operate the ultrasound transducer system to increase permeability of tissue at the target region, and the tissue disruption is the increase in permeability. In some implementations, the tissue is a blood-brain barrier. In some implementations, the one or more processors are configured to cause MRI contrast agent to be administered so it is present at the target region when the second device is operated to obtain the image.

In some implementations, the one or more processors are configured to operate a source of ultrasound contrast agent including a suspension of microbubbles and a source of MRI contrast agent so that the ultrasound contrast agent and the MRI contrast agent are present at the target region when the second device is operated to obtain the image.

In some implementations, the one or more processors are configured to operate the first device to record signals from tissue at the target region and to detect the location of the tissue disruption based at least in part on the signals recorded by the first device.

In some implementations, the one or more processors are configured to operate the first device to generate a second image of the target region from the signals recorded by the first device, and detect the location of the tissue disruption in the second image.

As noted earlier, the approach described above may be applied to any two or more imaging modalities that can resolve different but co-located tissue events. For example, PET biomarkers that ordinarily do not penetrate the BBB may be administered; once the BBB is opened at one or more specific locations as described above, passage of the PET biomarker can be detected to permit registration of the PET and ultrasound (or other) system. Suitable PET biomarkers include [68Ga]DTPA, 2-amino-[3-11C]isobutyric acid, and Gd nanoparticles.

Moreover, it is to be understood that the features of the various embodiments described herein are not necessarily mutually exclusive and can exist in various combinations and permutations, even if such combinations or permutations are not made express herein. In fact, variations, modifications, and other implementations of what is described herein will occur to those of ordinary skill in the art.

In general, functionality for evaluating and/or computing an imaging registration between two or more imaging systems, as well as controlling the operation of these systems, may be structured in one or more modules implemented in hardware, software, or a combination of both. For embodiments in which the functions are provided as one or more software programs, the programs may be written in any of a number of high level languages such as FORTRAN, PASCAL, JAVA, C, C++, C#, BASIC, various scripting languages, and/or HTML. Additionally, the software can be implemented in an assembly language directed to the microprocessor resident on a target computer; for example, the software may be implemented in Intel 80x86 assembly language if it is configured to run on an IBM PC or PC clone. The software may be embodied on an article of manufacture including, but not limited to, a floppy disk, a jump drive, a hard disk, an optical disk, a magnetic tape, a PROM, an EPROM, EEPROM, field-programmable gate array, or CD-ROM. Embodiments using hardware circuitry may be implemented using, for example, one or more FPGA, CPLD or ASIC processors.

## Claims

1. A system for registering first and second devices affecting internal anatomic tissue, the system comprising one or more processors (116, 146, 156) configured to:
operate the first device to:
create a tissue disruption at a target region in the internal anatomic tissue; and
detect a location of the tissue disruption in the target region;
operate the second device to:
obtain an image of the target region; and
detect a location of the tissue disruption in the image;
register a first coordinate reference frame of the first device and a second coordinate reference frame of the second device to a common coordinate reference frame based on (i) the location of the tissue disruption in the target region detected by the first device and (ii) the location of the tissue disruption in the image detected by the second device.

2. The system of claim 1, wherein the first device is an ultrasound transducer system (150, 205) and the second device is a magnetic-resonance imaging, MRI, system (102, 210).

3. The system of claim 1 or claim 2, wherein the one or more processors (116, 146, 156) are configured to register the first and second coordinate reference frames to the common coordinate reference frame by assigning the same location in each of the first and second coordinate reference frames to the tissue disruption in the target region.

4. The system of claim 1 or claim 2, wherein the one or more processors (116, 146, 156) are configured to register the first and second coordinate reference frames to the common coordinate reference frame by establishing a coordinate transformation to relate one of the first and second coordinate reference frames to the other of the first and second coordinate reference frames.

5. The system of claim 4, wherein the one or more processors (116, 146, 156) are configured to operate one of the first and second devices to communicate the coordinate transformation to the other of the first and second devices.

6. The system of any of claims 2-5, wherein the tissue disruption is visualizable by MRI and/or computationally detectable in a T2 MR image or in a T2* MR image.

7. The system of any of claims 2-6, wherein the tissue disruption is a transient disruption.

8. The system of any of claims 2-7, wherein the one or more processors (116, 146, 156) are configured to operate the ultrasound transducer system (150, 205) to induce acoustic cavitation at the target region, and the tissue disruption is mechanically ruptured tissue caused by the acoustic cavitation.

9. The system of any of claims 2-7, wherein the one or more processors (116, 146, 156) are configured to (i) operate a source of ultrasound contrast agent including a suspension of microbubbles and (ii) cause the ultrasound contrast agent to be administered such that the ultrasound contrast agent reaches the target region before the first device is operated to create the tissue disruption at the target region.

10. The system of any of claims 2-7, wherein the one or more processors (116, 146, 156) are configured to operate the ultrasound transducer system (150) to increase permeability of tissue at the target region, and the tissue disruption is the increase in permeability.

11. The system of claim 10, wherein the tissue is a blood-brain barrier.

12. The system of claim 11, wherein the one or more processors (116, 146, 156) are configured to cause MRI contrast agent to be administered so it is present at the target region when the second device is operated to obtain the image.

13. The system of any of claims 2-7, wherein the one or more processors (116, 146, 156) are configured to operate a source of ultrasound contrast agent including a suspension of microbubbles and a source of MRI contrast agent so that the ultrasound contrast agent and the MRI contrast agent are present at the target region when the second device is operated to obtain the image.

14. The system of any of claims 1-13, wherein the one or more processors (116, 146, 156) are configured to operate the first device to record signals from tissue at the target region and to detect the location of the tissue disruption based at least in part on the signals recorded by the first device.

15. The system of claim 14, wherein the one or more processors (116, 146, 156) are configured to operate the first device to generate a second image of the target region from the signals recorded by the first device, and detect the location of the tissue disruption in the second image.

## Patentansprüche

1. System zum Eintragen einer ersten und einer zweiten Vorrichtung, die sich auf inneres anatomisches Gewebe auswirken, das System umfassend einen oder mehrere Prozessoren (116, 146, 156), die dazu konfiguriert sind:
die erste Vorrichtung dazu zu betreiben:
eine Gewebedurchtrennung in einer Zielregion in dem inneren anatomischen Gewebe herzustellen; und
einen Ort der Gewebedurchtrennung in der Zielregion zu erkennen;
die zweite Vorrichtung dazu zu betreiben:
ein Bild der Zielregion zu erhalten; und
einen Ort der Gewebedurchtrennung in dem Bild zu erkennen;
ein erstes Koordinatenreferenz-Frame der ersten Vorrichtung und ein zweites Koordinatenreferenz-Frame der zweiten Vorrichtung in einem gemeinsamen Koordinatenreferenz-Frame auf Basis von Folgendem einzutragen: (i) dem von der ersten Vorrichtung erkannten Ort der Gewebedurchtrennung in der Zielregion und (ii) dem von der zweiten Vorrichtung erkannten Ort der Gewebedurchtrennung in dem Bild.

2. System nach Anspruch 1, wobei die erste Vorrichtung ein Ultraschallwandlersystem (150, 205) ist und die zweite Vorrichtung ein Magnetresonanzbildgebungs-, MRI-, System (102, 210) ist.

3. System nach Anspruch 1 oder Anspruch 2, wobei der eine oder die mehreren Prozessoren (116, 146, 156) dazu konfiguriert sind, das erste und das zweite Koordinatenreferenz-Frame in dem gemeinsamen Koordinatenreferenz-Frame durch Zuweisen desselben Ortes in jedem von dem ersten und dem zweiten Koordinatenreferenz-Frame zu der Gewebedurchtrennung in der Zielregion einzutragen.

4. System nach Anspruch 1 oder Anspruch 2, wobei der eine oder die mehreren Prozessoren (116, 146, 156) dazu konfiguriert sind, das erste und das zweite Koordinatenreferenz-Frame in dem gemeinsamen Koordinatenreferenz-Frame durch Festlegen einer Koordinatentransformation einzutragen, um eines von dem ersten und dem zweiten Koordinatenreferenz-Frame mit dem anderen von dem ersten und dem zweiten Koordinatenreferenz-Frame in Bezug zu setzen.

5. System nach Anspruch 4, wobei der eine oder die mehreren Prozessoren (116, 146, 156) dazu konfiguriert sind, eine von der ersten und der zweiten Vorrichtung dazu zu betreiben, die Koordinatentransformation an die andere von der ersten und der zweiten Vorrichtung zu kommunizieren.

6. System nach einem der Ansprüche 2 bis 5, wobei die Gewebedurchtrennung durch MRI visualisierbar und/oder rechnerisch in einem T2-MR-Bild oder in einem T2*-MR-Bild erkennbar ist.

7. System nach einem der Ansprüche 2 bis 6, wobei die Gewebedurchtrennung eine vorübergehende Durchtrennung ist.

8. System nach einem der Ansprüche 2 bis 7, wobei der eine oder die mehreren Prozessoren (116, 146, 156) dazu konfiguriert sind, das Ultraschallwandlersystem (150, 205) dazu zu betreiben, eine akustische Kavitation in der Zielregion herbeizuführen, und die Gewebedurchtrennung mechanisch durchtrenntes Gewebe ist und durch die akustische Kavitation verursacht wurde.

9. System nach einem der Ansprüche 2 bis 7, wobei der eine oder die mehreren Prozessoren (116, 146, 156) dazu konfiguriert sind, (i) eine Quelle von Ultraschallkontrastmittel zu betreiben, die eine Suspension aus Mikrobläschen beinhaltet, und (ii) zu verursachen, dass das Ultraschallkontrastmittel derart abgegeben wird, dass das Ultraschallkontrastmittel die Zielregion erreicht, bevor die erste Vorrichtung dazu betrieben wird, die Gewebedurchtrennung in der Zielregion herzustellen.

10. System nach einem der Ansprüche 2 bis 7, wobei der eine oder die mehreren Prozessoren (116, 146, 156) dazu konfiguriert sind, das Ultraschallwandlersystem (150) dazu zu betreiben, eine Gewebepermeabilität in der Zielregion zu erhöhen und die Gewebedurchtrennung die Erhöhung der Permeabilität darstellt.

11. System nach Anspruch 10, wobei das Gewebe eine Blut-Hirn-Schranke ist.

12. System nach Anspruch 11, wobei der eine oder die mehreren Prozessoren (116, 146, 156) zu konfiguriert sind, zu verursachen, dass ein MRI-Kontrastmittel so abgegeben wird, dass es in der Zielregion vorhanden ist, wenn die zweite Vorrichtung dazu betrieben wird, das Bild zu erhalten.

13. System nach einem der Ansprüche 2 bis 7, wobei der eine oder die mehreren Prozessoren (116, 146, 156) dazu konfiguriert sind, eine Quelle von Ultraschallkontrastmittel, die ein Suspension von Mikrobläschen beinhaltet, und eine Quelle von MRI-Kontrastmittel so zu betreiben, dass das Ultraschallkontrastmittel und das MRI-Kontrastmittel in der Zielregion vorhanden sind, wenn die zweite Vorrichtung dazu betrieben wird, das Bild zu erhalten.

14. System nach einem der Ansprüche 1 bis 13, wobei der eine oder die mehreren Prozessoren (116, 146, 156) dazu konfiguriert sind, die erste Vorrichtung dazu zu betreiben, Signale von Gewebe in der Zielregion aufzuzeichnen und den Ort der Gewebedurchtrennung mindestens teilweise auf Basis der durch die erste Vorrichtung aufgezeichneten Signale zu erkennen.

15. System nach Anspruch 14, wobei der eine oder die mehreren Prozessoren (116, 146, 156) dazu konfiguriert sind, die erste Vorrichtung dazu zu betreiben, ein zweites Bild der Zielregion aus den von der ersten Vorrichtung aufgezeichneten Signalen zu erzeugen und den Ort der Gewebedurchtrennung in dem zweiten Bild zu erkennen.

## Revendications

1. Système de recalage de premier et deuxième dispositifs affectant un tissu anatomique interne, le système comprenant un ou plusieurs processeurs (116, 146, 156) configurés pour :
faire fonctionner le premier dispositif pour :
créer une rupture tissulaire au niveau d'une région cible dans le tissu anatomique interne ; et
détecter un emplacement de la rupture tissulaire dans la région cible ;
faire fonctionner le deuxième dispositif pour :
obtenir une image de la région cible ; et
détecter un emplacement de la rupture tissulaire dans l'image ;
recaler un premier référentiel de coordonnées du premier dispositif et un deuxième référentiel de coordonnées du deuxième dispositif sur un référentiel de coordonnées commun sur la base de (i) l'emplacement de la rupture tissulaire dans la région cible détecté par le premier dispositif et de (ii) l'emplacement de la rupture tissulaire dans l'image détecté par le deuxième dispositif.

2. Système selon la revendication 1, dans lequel le premier dispositif est un système de transducteur ultrasonore (150, 205) et le deuxième dispositif est un système d'imagerie par résonance magnétique, IRM, (102, 210).

3. Système selon la revendication 1 ou la revendication 2, dans lequel les un ou plusieurs processeurs (116, 146, 156) sont configurés pour recaler les premier et deuxième référentiels de coordonnées sur le référentiel de coordonnées commun en attribuant le même emplacement sur chacun des premier et deuxième référentiels de coordonnées à la rupture tissulaire dans la région cible.

4. Système selon la revendication 1 ou la revendication 2, dans lequel les un ou plusieurs processeurs (116, 146, 156) sont configurés pour recaler les premier et deuxième référentiels de coordonnées sur le référentiel de coordonnées commun en établissant une transformation de coordonnées pour relier l'un des premier et deuxième référentiels de coordonnées à l'autre des premier et deuxième référentiels de coordonnées.

5. Système selon la revendication 4, dans lequel les un ou plusieurs processeurs (116, 146, 156) sont configurés pour faire fonctionner l'un des premier et deuxième dispositifs pour communiquer la transformation de coordonnées à l'autre des premier et deuxième dispositifs.

6. Système selon l'une quelconque des revendications 2 à 5, dans lequel la rupture tissulaire est visualisable par IRM et/ou détectable informatiquement dans une image IRM pondérée en T2 ou dans une image IRM pondérée en T2*.

7. Système selon l'une quelconque des revendications 2 à 6, dans lequel la rupture tissulaire est une rupture transitoire.

8. Système selon l'une quelconque des revendications 2 à 7, dans lequel les un ou plusieurs processeurs (116, 146, 156) sont configurés pour faire fonctionner le système de transducteur ultrasonore (150, 205) pour induire une cavitation acoustique au niveau de la région cible, et la rupture tissulaire est une rupture mécanique du tissu provoquée par la cavitation acoustique.

9. Système selon l'une quelconque des revendications 2 à 7, dans lequel les un ou plusieurs processeurs (116, 146, 156) sont configurés pour (i) faire fonctionner une source d'agent de contraste ultrasonore incluant une suspension de microbulles et (ii) amener l'agent de contraste ultrasonore à être administré de telle sorte que l'agent de contraste ultrasonore atteigne la région cible avant que le premier dispositif soit actionné pour créer la rupture tissulaire au niveau de la région cible.

10. Système selon l'une quelconque des revendications 2 à 7, dans lequel les un ou plusieurs processeurs (116, 146, 156) sont configurés pour faire fonctionner le système de transducteur ultrasonore (150) pour augmenter la perméabilité du tissu au niveau de la région cible, et la rupture tissulaire est l'augmentation de la perméabilité.

11. Système selon la revendication 10, dans lequel le tissu est une barrière hémato-encéphalique.

12. Système selon la revendication 11, dans lequel les un ou plusieurs processeurs (116, 146, 156) sont configurés pour amener l'agent de contraste IRM à être administré afin qu'il soit présent au niveau de la région cible lorsque le deuxième dispositif est mis en fonctionnement pour obtenir l'image.

13. Système selon l'une quelconque des revendications 2 à 7, dans lequel les un ou plusieurs processeurs (116, 146, 156) sont configurés pour faire fonctionner une source d'agent de contraste ultrasonore incluant une suspension de microbulles et une source d'agent de contraste IRM afin que l'agent de contraste ultrasonore et l'agent de contraste IRM soient présents au niveau de la région cible lorsque le deuxième dispositif est mis en fonctionnement pour obtenir l'image.

14. Système selon l'une quelconque des revendications 1 à 13, dans lequel les un ou plusieurs processeurs (116, 146, 156) sont configurés pour faire fonctionner le premier dispositif pour enregistrer des signaux provenant du tissu au niveau de la région cible et pour détecter l'emplacement de la rupture tissulaire sur la base au moins en partie des signaux enregistrés par le premier dispositif.

15. Système selon la revendication 14, dans lequel les un ou plusieurs processeurs (116, 146, 156) sont configurés pour faire fonctionner le premier dispositif pour générer une deuxième image de la région cible à partir des signaux enregistrés par le premier dispositif et détecter l'emplacement de la rupture tissulaire dans la deuxième image.
